(19) Europäisches Patentamt
European Patent Office
Office européen des brevets

(11) Numéro de publication: **0 197 868**
**B1**

(12) # FASCICULE DE BREVET EUROPÉEN

(45) Date de publication du fascicule du brevet: **18.05.88**

(51) Int. Cl.⁴: **C 07 C 43/225**, C 07 C 149/34, C 07 C 41/22

(21) Numéro de dépôt: **86420091.0**

(22) Date de dépôt: **27.03.86**

(54) **Procédé de préparation de dérivés dichlorotrifluoroéthoxy et éthylthioaromatiques.**

(30) Priorité: **29.03.85 FR 8504757**

(43) Date de publication de la demande: **15.10.86 Bulletin 86/42**

(45) Mention de la délivrance du brevet: **18.05.88 Bulletin 88/20**

(84) Etats contractants désignés: **AT BE CH DE FR GB IT LI LU NL SE**

(56) Documents cité: **DE-B-1 183 096**

(73) Titulaire: **RHONE- POULENC CHIMIE, 25, quai Paul Doumer, F-92408 Courbevoie Cédex (FR)**

(72) Inventeur: **Langlois, Bernard, 91, rue Duguesclin, F-69006 Lyon (FR)**

(74) Mandataire: **Le Pennec, Magali, RHONE- POULENC INTERSERVICES Service Brevets Chimie 25, Quai Paul Doumer, F-92408 Courbevoie Cédex (FR)**

**EP 0 197 868 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention concerne un procédé général de préparation de dérivés dichlorotrifluoroéthoxy et éthylthioaromatiques et tout particulièrement un procédé de préparation de dérivés 1,1-dichloro 2,2,2-trifluoroéthoxyaromatiques et 1,1-dichloro 2,2,2 trifluoroéhylthioaromatiques.

Le premier procédé connu pour préparer des produits de ce type est décrit par "R.F. Clark et J.H. Simons (J. Org. Chem., 1961, 26, 5 197) sur un seul exemple qui consiste à faire réagir le pentachlorure de phosphore sur le trifluoroacétate de phényle. La préparation de ce dernier et la manipulation du pentachlorure de phosphore ne sont pas, à l'échelle industrielle, faciles et le rendement annoncé est faible (43 %).

Le second procédé connu est décrit dans l'exemple 7 du brevet allemand N° 1 183 096.

Dans ce brevet, l'inventeur cherche à obtenir des dérivés α, β perhalogénés et particulièrement des dérivés α,α -difluorés β,β,β- trichlorés; dans l'exemple 7 en faisant réagir le chlorure de 3-(β,β,β -trifluoroéthoxy)benzoyle avec du chlore et du trichlorure de phosphore en présence de radiations, il obtient du chlorure de 3-(α,α-dichloro β,β,β -trifluoroéthoxy)benzoyle. Il faut noter que le noyau aromatique ne comporte dans cet exemple que des groupements électroattracteurs.

La chloration en présence de ce système catalytique ($PCl_3$ et radiations) est particulièrement longue.

La présente invention a su vaincre ces inconvénients et a pour objet un procédé de préparation de dérivés 1,1-dichloro 2,2,2-trifluoroéthoxyaromatiques et de dérivés 1,1-dichloro 2,2,2-trifluoro-éthylthioaromatiques caractérisé en ce qu'on met en présence un dérivé 2,2,2-trifluoroéthoxyaromatiques ou 2,2,2-trifluoroéthylthioaromatique avec du chlore en présence de rayonnements dont les longueurs d'ondes sont comprises de préférence entre 250 et 600 nm et en présence éventuellement d'un solvant.

Les composés de départ repondent notamment à la formule générale suivante (I):

$$R_pAr\ (ACH_2CF_3)_n \qquad (I)$$

dans laquelle:

- Ar représente un noyau aromatique mono ou polycyclique
- A représente le soufre ou l'oxygène
- n représente un nombre entier supérieur ou égal à 1 et inférieur ou égal à 3.
- R représente au moins un substituant choisi parmi le groupe comprenant les radicaux hydrogène, alkyle, alcoxy, alkylthio, halogéno, halogénoalkyle, halogénoalcoxy, halogénoalkylthio, phényle, phénoxy, benzoyle, alcoxycarbonyle, halogénocarbonyle, cyano, nitro.
- p est un nombre entier égal ou supérieur à 1 et égal ou inférieur à 5.

On préfère utiliser les produits de formule (I) dans lesquels n est égal à 1 ou 2 et Ar est un novau benzénique.

L'acidité des atomes d'hydrogène situés en du groupe trifluorométhyle est fortement augmentée par l'effet attracteur dudit groupe, aussi n'était-il pas évident de pouvoir procéder à une photochloration.

L'étape de chloration photochimique peut être réalisée en présence ou en l'absence de solvant.

Les solvants qui peuvent être mis en heuvre sont des solvants résistant à la chloration et particulièrement choisis parmi les chlorobenzènes liquides et le tétrachlorure de carbone.

On préfère tout particulièrement utiliser le tétrachlorure de carbone.

Lorsque le composé de départ est subtitué sur le noyau par un groupe électrodonneur ou hydrogène, la réaction a lieu de préférence en présence d'un solvant lorsqu'on ne veut pas obtenir de chloration supplémentaire du noyau. On entend au sens de la présente invention par groupe électrodonneur, les éléments choisis mais non limitativement parmi les groupes alkyle, alcoxy, alkylthio, phénoxy, phényle. Quand le composé de départ est liquide et qu'il est substitué sur le noyau par un groupe électroattracteur, la réaction a lieu de préférence en l'absence d'un solvant.

La réaction a lieu de préférence, à la pression atmosphérique mais, dans le cas de composé de départ substitués sur le noyau par un groupe électroattracteur, une pression supérieure est avantageuse. La pression sera simplement adaptée à l'économie du procédé.

La température de la réaction est comprise de préférence entre 70 et 200° C et tout particulièrement entre 70 et 85° C lorsque l'on opère en présence de tétrachlorure de carbone.

Les temps de réaction sont généralement compris entre 2 heures et 40 heures.

Les rayonnements sont obtenus généralement par utilisation d'un tube à décharge contenant des gaz rares et/ou de la vapeur de mercure.

Les produits obtenus selon le procédé de l'invention répondent notamment à la formule générale (II):

$$(R_1)_p\ Ar(ACCl_2CF_3)_n \qquad (II)$$

dans laquelle:

- Ar, n, p, A ont la signification précédente
- $R_1$ représente au moins un élément choisi dans le groupe comprenant les radicaux hydrogène, halogéno, halogénoalkyle, halogénoalcoxy, halogénoalkylthio, phényle, phénoxy, benzoyle, halogénoalcoxycarbonyle, halogénocarbonyle, cyano.

On peut citer comme exemples de composés pouvant être préparés par le procédé selon l'invention, le 1,1-dichloro 2,2,2-trifluoroéthoxybenzène, les 4'chloro, 2'chloro, 4'fluoro, 2'fluoro, 4'trichlorométhyl,

4'trichlorométhoxy, 4'trichlorométhylthio, 4'penta-chloroéthyl, 3'trifluorométhyl, 4'trifluorométhyl, 1,1-dichloro 2,2,2-trifluoroéthoxybenzènes, les bis (1,1-dichloro 2,2,2 trifluoroéthoxy)benzènes, le chlorure de 1,1-dichloro 2,2,2-trifluoro-éthoxybenzoyle, le 1,1-dichloro 2,2,2-trifluoroéthoxynaphtalène, le 1,1- dichloro 2,2,2-trifluoroéthylthiobenzène, les 4' chloro, 3' chloro, 4' nitro, 1,1-dichloro 2,2,2-trifluoroéthylthiobenzènes.

Les composés de la présente invention sont utilisés comme intermédiaires de synthèse pour la préparation de dérivés à activité pharmaceutique, vétérinaire ou phytosanitaire et dans l'industrie des lubrifiants (US 4 366 168).

L'invention va être plus complètement décrite à l'aide des exemples qui vont auivre. Ces derniers ne devront être considérés en aucun cas comme limitant de façon quelconque l'invention.

**Exemple 1**

Synthèse du chloro-4(dichloro-1,1 trifluoro-2,2,2 éthoxy)benzène
Au sein du tétrachlorure de carbone en présence de radiations

Dans un réacteur cylindrique de deux litres utiles, muni d'une agitation, d'un réfrigérant ascendant, d'un dispositif de mesure de température, d'un tube plongeant poreux pour introduction de gaz et d'une gaine centrale dans laquelle est placée une lampe à décharge émettant autour de 400 nm, on charge deux litres d'une solution contenant 379 g (1,8 mole) de chloro-4(trifluoro-2,2,2 éthoxy)benzène pur dans du tétrachlorure de carbone.

On porte la solution irradiée au reflux sous courant d'azote. Une fois le reflux atteint, on arrête l'introduction d'azote et on la remplace par une introduction de chlore gazeux (débit initial = 520 g/h) de manière à saturer de chlore la solution bouillante irradiée.

La chloration photochimique dure 3 h 30 minutes. Son évolution est suivie par chromatographie en phase gazeuse.

Lorsqu'elle est terminée, on maintient le milieu réactionnel au reflux sous irradiation et on remplace le chlore par de l'azote jusqu'à dégazage complet du liquide. On refroidit et on arrête l'irradiation.

On distille le tétrachlorure de carbone sous pression atmosphérique puis le chloro-4(dichloro-1,1 trifluoro-2,2,2 éthoxy)benzène sous pression réduite ($Eb_{18}$ = 103°C).

On recueille ainsi 463 g de produit représentant un rendement de 92 %.

**Exemple 2**

En l'absence de solvant et en présence de radiations

La même réaction effectuée suivant le processus ci-dessus, mais à 150°C en l'absence de tétrachlorure de carbone, conduit, en 2 h 30 minutes à un rendement en chloro-4(dichloro-1,1 trifluoro-2,2,2 éthoxy)benzène distillé de 84 %.

**Exemples 3 à 16**

Ils sont résumés dans le tableau suivant:

**TABLEAU I**

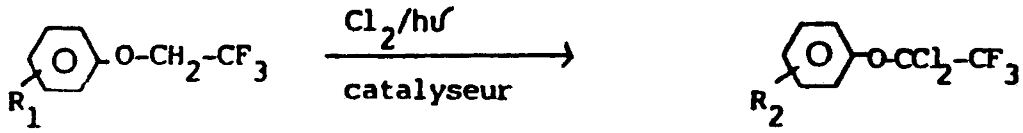


| Essai | $R_1$ | Solvant | $[Ar\text{-}O\text{-}CH_2CF_3]$ | T°C | Durée | $R_2$ | Rdt distillé |
|---|---|---|---|---|---|---|---|
| 3 | C1-2 | sans | - | 150 | 4 h | C1-2 | 75 % |
| 4 | H | $CCl_4$ | 2 moles/l | 78-81 | 24 h | H | 61 % |
| 5 | $CH_3$-4 | TCR | 1,14 moles/l | 115 | 9 h | $CCl_3$-4 | 45 % |
| 6 | $CH_3O$-4 | $CCl_4$ | 1 mole/l | 78-80 | 8 h | $CCl_3O$-4 | 44 % |
| 7 | $CF_3CH_2$-O-4 | $CCl_4$ | 0,5 mole/l | 78-80 | 6 h | $CF_3$-$CCl_2$-O-4 | 58 % |
| 8 | $CF_3$-4 | sans | - | 120-130 | 5 h | $CF_3$-4 | 73 % |
| 9 | NC-4 | $CCl_4$ | 1 mole/l | 80 | 5 h | NC-4 | 64 % |
| 10 | $CH_3O_2C$-4 | ODCB | 3 moles/l | 160-170 | 12 h | C10C-4 | 85% |
| 11 | $CH_3O_2C$-2 | ODCB | 3 moles/l | 160-170 | 16 h | ClOC-2 | 65% |
| 12 | $pCF_3CH_2OC_6H_4CO$-4 | $CCl_4$ | 0,5 mole/l | 78-80 | 20 h | p-$CF_3$-$CCl_2OC_6H_4$-CO-4 | 54 % |
| 13 | $C_{10}H_7OCH_2CF_3$-2 | $CCl_4$ | 1 mole/l | 80 | 20 h | $C_{10}H_7OCCl_2CF_3$-2 | 57 % |
| 14 | H | $CCl_4$ | 1 mole/l | 78-80°C | 20 h | H | 61 % |
| 15 | Cl-3 | $CCl_4$ | 1 mole/l | 78-80°C | 19 h | Cl-3 | 56 % |
| 16 | $O_2N$-4 | $CCl_4$ | 1 mole/l | 78-80°C | 23h30 | $O_2N$-4 | 90 % |

TCB = Trichlorobenzène-1 2 4 ODCB = orthodichlorobenzène

**Essai 17**

Lorsqu'on soumet le (trifluoro-2,2,2 éthoxy)benzène à une chloration effectuée dans les conditions de l'essai 2 mais en l'absence de solvant et à 110°C-120°C, la première réaction observée est une chloration du noyau aromatique qui précède celle du substituant trifluoroéthoxy: au bout de deux heures, la composition molaire de la masse réactionnelle est la suivante:

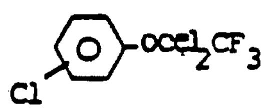 54 %

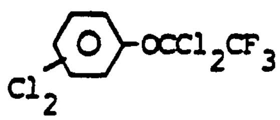 13 %

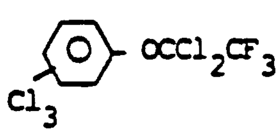 2 %

impuretés lourdes 31 %

impuretés lourdes 31 %

Un solvant est donc nécessaire pour obtenir sélectivement le (dichloro-1,1 trifluoro-2,2,2 éthoxy)benzène à partir de (trifluoro-2,2,2 éthoxy)benzène (cf. essai 4).

**Revendications**

1. Procédé de préparation de dérivés 1,1-dichloro 2,2,2-tri-fluoroéthoxyaromatiques ou 1,1-dichloro 2,2,2-trifluoroéthylthioaromatiques caractérisé en ce qu'on met en contact un dérivé 2,2,2-trifluoroéthoxyaromatique ou 2,2,2-trifluoroéthylthioaromatique avec du chlore en présence de rayonnements ayant une longueur d'onde comprise entre 250 et 600nm.

2. Procédé selon la revendication 1, caractérisé en ce que les dérivés 2,2,2-trifluoroéthoxyaromatiques ou 2,2,2-trifluoroéthyl-thioaromatiques répondent à la formule générale (I):

$$R_p\,Ar(ACH_2CF_3)_n \qquad (I)$$

dans laquelle:
- Ar représente un noyau aromatique mono ou polycyclique.
- n est un nombre entier supérieur ou égal à 1 et inférieur ou égal à 3.
- R représente au moins un substituant choisi dans le groupe comprenant les radicaux hydrogène, alkyle, alcoxy, alkylthio halogéno, halogénoalkyle, halogénoalcoxy, halogénoalkylthio, phényle, phénoxy, benzoyle, alcoxycarbonyle, halogénocarbonyle, cyano.
- A représente le soufre ou l'oxygène.
- p est un nombre entier égal ou supérieur à i et égal ou inférieur à 5.

3. Procédé selon la revendication 2 caractérisé en ce que dans la formule (I) n = 1 ou 2.

4. Procédé selon le revendication 1 caractérisé en ce que la chloration est effectuée en présence de solvants choisis parmi les chlorobenzènes et le tétrachlorure de carbone.

5. Procédé selon la revendication 4 caractérisé en ce que le solvant est le tétrachlorure de carbone.

6. Procédé selon la revendication 1 caractérisé en ce que la chloration a lieu à une température comprise entre 70 et 200° C.

7. Procédé selon les revendications 5 et 6 caractérisé en ce que la température de réaction est comprise entre 70 et 85° C.

**Patentansprüche**

1. Verfahren zur Herstellung der Derivate von 1,1-Dichlor-2,2,2-trifluorethoxyaromaten oder 1,1-Di-chlor-2,2,2-trifluorethylthioaromaten, dadurch gekennzeichnet, daß man ein aromatisches 2,2,2-Trifluorethoxyderivat oder ein aromatisches 2,2,2-Trifluorethylthioderivat mit Chlor unter Bestrahlung einer Wellenlänge zwischen 250 und 600 nm in Kontakt bringt.

2. Verfahren nach Anspruch 1, dadurch gekennzeichnet, daß die aromatischen 2,2,2-Trifluorethoxyderivate oder aromatischen 2,2,2-Trifluorethylthioderivate der allgemeinen Formel (I) genügen:

$$R_pAr(ACH_2CF_3)_n \qquad (I)$$

in der:
- Ar einen mono- oder polycyclischen aromatischen Kern darstellt
- n eine ganze Zahl ist, die größer oder gleich 1 und kleiner oder gleich 3 ist
- R wenigstens einen Substituenten darstellt, der aus der Gruppe ausgewählt ist, die die Radikale Wasserstoff, Alkyl, Alkoxy, Alkylthio, Halogen, Halogenalkyl, Halogenalkoxy, Halogenalkylthio, Phenyl, Phenoxy, Benzoyl, Alkoxycarbonyl, Halogencarbonyl, Cyano umfaßt
- A Schwefel oder Sauerstoff bedeutet
- p eine ganze Zahl ist, die größer oder gleich 1 und kleiner oder gleich 5 ist.

3. Verfahren nach Anspruch 2, dadurch gekennzeichnet, daß in Formel (I) n = 1 oder 2 ist.

4. Verfahren nach Anspruch 1, dadurch gekennzeichnet, daß die Chlorierung in Gegenwart von Lösemitteln durchgeführt wird, die unter den Chlorbenzolen und Tetrachlorkohlenstoff ausgewählt werden.

5. Verfahren nach Anspruch 4, dadurch gekennzeichnet, daß das Lösemittel Tetrachlorkohlenstoff ist.

8. Verfahren nach Anspruch 1, dadurch gekennzeichnet, daß die Chlorierung bei einer Temperatur zwischen 70 und 200° C stattfindet.

7. Verfahren nach den Ansprüchen 5 und 6, dadurch gekennzeichnet, daß die Reaktionstemperatur zwischen 70 und 85° C liegt.

**Claims**

1. Process for the preparation of 1,1-dichloro-2,2,2-trifluoroethoxyaromatic or 1,1-di-chloro-2,2,2-trifluoroethylthioaromatic derivatives, characterized in that a 2,2,2-trifluoroethoxyaromatic or a 2,2,2-trifluoroethylthioaromatic derivative is brought into contact with chlorine in the presence of radiations of a

wavelength between 250 and 600 nm.

2. Process according to claim 1, characterized in that the 2,2,2-trifluoroethoxyaromatic or 2,2,2-trifluoroethylthioaromatic derivatives correspond to the general formula (I):

$$R_pAr(ACH_2CF_3)_n \quad (I)$$

in which formula:
- Ar denotes a monocyclic or polycyclic aromatic nucleus,
- n is an integer greater than or equal to 1 and smaller than or equal to 3,
- R denotes at least one substituent chosen from the group comprising the hydrogen, alkyl, alkoxy, alkylthio, halo, haloalkyl, haloalkoxy, haloalkylthio, phenyl, phenoxy, benzoyl, alkoxycarbonyl, halocarbonyl and cyano radicals,
- A denotes sulphur or oxygen, and
- p is an integer equal to or greater than 1 and equal to or smaller than 5.

3. Process according to claim 2, characterized in that in formula (I), n = 1 or 2.

4. Process according to claim 1 characterized in that the chlorination is carried out in the presence of solvents chosen from chlorobenzenes and carbon tetrachloride.

5. Process according to claim 4, characterized in that the solvent is carbon tetrachloride.

6. Process according to claim 1, characterized in that the chlorination takes place at a temperature between 70 and 200° C.

7. Process according to claims 5 and 6, characterized in that the reaction temperature is between 70 and 85° C.